(19) **Europäisches Patentamt / European Patent Office / Office européen des brevets**

(11) **EP 4 494 758 A1**

(12) **EUROPEAN PATENT APPLICATION**
published in accordance with Art. 153(4) EPC

(43) Date of publication:
**22.01.2025  Bulletin 2025/04**

(21) Application number: **23770870.6**

(22) Date of filing: **16.03.2023**

(51) International Patent Classification (IPC):
*B01J 20/22 (2006.01)*   *B01D 53/28 (2006.01)*
*C07C 57/15 (2006.01)*   *C07C 63/24 (2006.01)*
*C07C 65/03 (2006.01)*   *C07C 229/76 (2006.01)*
*C07F 1/08 (2006.01)*   *C07F 5/06 (2006.01)*
*C07F 7/28 (2006.01)*

(52) Cooperative Patent Classification (CPC):
**B01D 53/28; B01J 20/22; C07C 57/15;**
**C07C 63/24; C07C 65/03; C07C 229/76;**
**C07F 1/08; C07F 5/06; C07F 7/28**

(86) International application number:
**PCT/JP2023/010263**

(87) International publication number:
**WO 2023/176917 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.03.2022  JP 2022042376**

(71) Applicants:
- **Kyoto University**
  **Kyoto-shi, Kyoto 606-8501 (JP)**
- **Sumitomo Chemical Company, Limited**
  **Tokyo 103-6020 (JP)**

(72) Inventors:
- **KITAGAWA, Susumu**
  **Kyoto-shi, Kyoto 606-8501 (JP)**
- **HIGUCHI, Masakazu**
  **Kyoto-shi, Kyoto 606-8501 (JP)**
- **OTAKE, Kenichi**
  **Kyoto-shi, Kyoto 606-8501 (JP)**
- **KIKUCHI, Yuta**
  **Ichihara-shi, Chiba 299-0195 (JP)**

(74) Representative: **Vossius & Partner**
**Patentanwälte Rechtsanwälte mbB**
**Siebertstrasse 3**
**81675 München (DE)**

(54) **METAL ORGANIC FRAMEWORK**

(57) An object of the present invention is to provide a MOF that exhibits greater than or equal to a predetermined adsorption amount at a high relative pressure while the amount of a target material to be adsorbed is rapidly increased according to the variation in relative pressure. The present invention is a metal organic framework, wherein on an adsorption-side isotherm of water vapor measured at 25°C, an inclination of a tangent line drawn at a point where a gradient of a curve is maximum is 7000 mL(STP)$\cdot g^{-1}$/relative pressure ($P/P_0$) or more, a water vapor adsorption amount at a relative pressure ($P/P_0$) of 0.96 is 440 mL(STP)$\cdot g^{-1}$ or more on the adsorption-side isotherm, and a vertical axis of the adsorption-side isotherm represents a water vapor adsorption amount and a horizontal axis of the adsorption-side isotherm represents a relative pressure ($P/P_0$).

[FIG. 1]

**Description**

TECHNICAL FIELD

The present invention relates to a metal organic framework.

BACKGROUND ART

Metal organic frameworks are also referred to as porous coordination polymers and are a class of materials which form porous structures by coordinate bonds between metal ions and organic ligands, and are expected to adsorb and desorb gas and expected to be applied to catalysts, and the like.

For example, Patent Literature 1 discloses a metal organic framework including metal ions, first ligands, second ligands, and optional third ligands. In the metal organic framework, the metal ion is an aluminum ion; the first and second ligands are each an ion of an organic compound that includes a hetero ring with two carboxyl groups; a heteroatom and the angle formed between the carboxyl groups satisfy a predetermined condition; the third ligand is an ion of an organic compound with two carboxyl groups; and the existing proportion of each of the first to third ligands is within a predetermined range.

CITATION LIST

PATENT LITERATURE

Patent Literature 1 : Japanese Laid-Open Patent Publication No.2020-176101

SUMMARY OF THE INVENTION

PROBLEMS TO BE SOLVED BY THE INVENTION

When the metal organic framework (hereinafter, may be referred to as MOF) is used to adsorb materials such as water, carbon dioxide, and hydrogen, the MOF sometimes needs to exhibit greater than or equal to a predetermined adsorption amount at a high relative pressure while the amount of a target material to be adsorbed is rapidly increased according to the variation in relative pressure. However, a MOF capable of satisfying such properties is not disclosed in the above-described Patent Literature 1.

Therefore, an object of the present invention is to provide a MOF that exhibits greater than or equal to a predetermined adsorption amount at a high relative pressure while the amount of a target material to be adsorbed is rapidly increased according to the variation in relative pressure.

SOLUTION TO THE PROBLEMS

The present invention that can achieve the problem is as follows.

[1] A metal organic framework, wherein

on an adsorption-side isotherm of water vapor measured at 25°C, an inclination of a tangent line drawn at a point where a gradient of a curve is maximum is 7000 mL(STP)$\cdot$g$^{-1}$/relative pressure (P/P$_0$) or more, a water vapor adsorption amount at a relative pressure (P/P$_0$) of 0.96 is 440 mL(STP)$\cdot$g$^{-1}$ or more on the adsorption-side isotherm, and a vertical axis of the adsorption-side isotherm represents a water vapor adsorption amount and a horizontal axis of the adsorption-side isotherm represents a relative pressure (P/P$_0$) (P represents water vapor pressure and P$_0$ represents saturation water vapor pressure).

[2] The metal organic framework according to [1], wherein the point where the gradient is maximum is in a range of relative pressures (P/P$_0$) of 0.1 to 0.4.
[3] The metal organic framework according to [1] or [2], wherein an adsorption amount ratio L on a side of low relative pressure obtained by formula (1) is 1.3 or more.

$$L=(A_{0.3}-A_{0.1})/(A_{0.9}-A_{0.3}) \ldots (1)$$

(in the formula, $A_{0.1}$ represents an adsorption amount $(mL(STP)\cdot g^{-1})$ at a relative pressure $(P/P_0)$ of 0.1, $A_{0.3}$ represents an adsorption amount $(mL(STP)\cdot g^{-1})$ at a relative pressure $(P/P_0)$ of 0.3, and $A_{0.9}$ represents an adsorption amount $(mL(STP)\cdot g^{-1})$ at a relative pressure $(P/P_0)$ of 0.9, on the adsorption-side isotherm)

[4] The metal organic framework according to any one of [1] to [3], wherein the adsorption amount at the relative pressure $(P/P_0)$ of 0.3 on the adsorption-side isotherm is 200 $mL(STP)\cdot g^{-1}$ or more.

[5] The metal organic framework according to any one of [1] to [4], wherein an amount of change in a water vapor adsorption amount in a section where the adsorption-side isotherm rises with an inclination of 1000 $mL(STP)\cdot g^{-1}$/relative pressure $(P/P_0)$ or more is 200 $mL(STP)\cdot g^{-1}$ or more.

[6] The metal organic framework according to any one of [1] to [5], wherein a relative pressure hysteresis ΔH obtained by formula (2) is within ±0.03.

$$\Delta H = AH - DH \ldots(2)$$

(in the formula, AH represents an average value $((P1+P2)/2)$ of a minimum relative pressure $(P/P_0)$ P1 and a maximum relative pressure $(P/P_0)$ P2 in a section where an inclination is 1000 $mL(STP)\cdot g^{-1}$/relative pressure $(P/P_0)$ or more on the adsorption-side isotherm, and DH represents an average value $((P3+P4)/2)$ of a minimum relative pressure $(P/P_0)$ P3 and a maximum relative pressure $(P/P_0)$ P4 in a section where an inclination is 1000 $mL(STP)\cdot g^{-1}$/relative pressure $(P/P_0)$ or more on a desorption-side isotherm)

[7] The metal organic framework according to any one of [1] to [6], wherein an adsorption amount hysteresis ΔA obtained by formula (3) is within ±30 $mL(STP)\cdot g^{-1}$.

$$\Delta A = AA - DA \ldots(3)$$

(in the formula, AA represents an average value $((A1+A2)/2)$ of an adsorption amount A1 $(mL(STP)\cdot g^{-1})$ at a minimum relative pressure $(P/P_0)$ and an adsorption amount A2 $(mL(STP)\cdot g^{-1})$ at a maximum relative pressure $(P/P_0)$ in a section where an inclination is 1000 $mL(STP)\cdot g^{-1}$/relative pressure $(P/P_0)$ or more on the adsorption-side isotherm, and DA represents an average value $((A3+A4)/2)$ of an adsorption amount A3 $(mL(STP)\cdot g^{-1})$ at a minimum relative pressure $(P/P_0)$ and an adsorption amount A4 $(mL(STP)g^{-1})$ at a maximum relative pressure $(P/P_0)$ in a section where an inclination is 1000 $mL(STP) \ g^{-1}$/relative pressure $(P/P_0)$ or more on a desorption-side isotherm)

[8] The metal organic framework according to any one of [1] to [7], wherein a metal ion constituting the metal organic framework is an ion of at least one metal selected from elements in groups 2 to 14 in the third to sixth periods in a periodic table.

[9] The metal organic framework according to any one of [1] to [8], wherein an organic ligand constituting the metal organic framework includes at least one selected from the group consisting of carboxylates represented by $R(COO^-)_n$ (R represents an n-valent group, and n represents an integer of 2 or more) and an oxalate ion $(COO^-)_2$.

ADVANTAGEOUS EFFECTS OF THE INVENTION

According to the present invention, it is possible to provide the MOF that exhibits greater than or equal to a predetermined adsorption amount at a high relative pressure while the amount of the target material to be adsorbed is rapidly increased according to the variation in relative pressure.

BREIF DESCRIPTION OF DRAWINGS

[FIG. 1] FIG. 1 is a graph showing adsorption and desorption isotherms of a MOF of Example 1.
[FIG. 2] FIG. 2 is a graph showing adsorption and desorption isotherms of a MOF of Comparative Example 2.

DESCRIPTION OF EMBODIMENTS

In the present invention, the adsorption and desorption properties of a MOF are evaluated by adsorption and desorption isotherms of water vapor measured at 25°C. The adsorption and desorption isotherms is a graph in which the vertical axis represents a water vapor adsorption amount $(mL(STP)\cdot g^{-1})$ per gram of the MOF and the horizontal axis represents a relative pressure $(P/P_0)$ (P represents water vapor pressure and $P_0$ represents saturation water vapor pressure). As for the MOF of the present invention, on the adsorption-side isotherm of water vapor measured at 25°C in which the vertical axis represents the water vapor adsorption amount and the horizontal axis represents the relative pressure ($P/P_0$), the inclination of a tangent line drawn at a point where the gradient of a curve is maximum is 7000 mL(STP)·g$^{-1}$/relative pressure ($P/P_0$) or more, and a water vapor adsorption amount at a relative pressure ($P/P_0$) of 0.96 is 440 mL(STP)·g$^{-1}$ or more.

On the adsorption-side isotherm of water vapor, the inclination of the tangent line drawn at the point where the gradient of the curve is maximum is preferably 8000 mL(STP)·g$^{-1}$/relative pressure ($P/P_0$) or more, more preferably 9000 mL(STP)·g$^{-1}$/relative pressure ($P/P_0$) or more, and still more preferably 14000 mL(STP)·g$^{-1}$/relative pressure ($P/P_0$) or more. The upper limit thereof is not particularly limited, and the inclination of the tangent line may be, for example, 50000 mL(STP)·g$^{-1}$/relative pressure ($P/P_0$) or less, or 40000 mL(STP)·g$^{-1}$/relative pressure ($P/P_0$) or less.

In addition, the water vapor adsorption amount at the relative pressure ($P/P_0$) of 0.96 is 440 mL(STP)·g$^{-1}$ or more, preferably 470 mL(STP)·g$^{-1}$ or more, and more preferably 500 mL(STP)·g$^{-1}$ or more. The upper limit thereof is not particularly limited, and the water vapor adsorption amount may be 800 mL(STP)·g$^{-1}$ or less, or 700 mL(STP)·g$^{-1}$ or less.

Furthermore, the MOF of the present invention also preferably satisfies any one or a combination of two or more of requirements (a) to (f) described below.

(a) Relative pressure ($P/P_0$) at a point where the gradient of an adsorption-side isotherm curve is maximum

The relative pressure ($P/P_0$) at the point where the gradient of the adsorption-side isotherm curve is maximum is preferably 0.1 to 0.4. In this case, a large amount of a target material such as water vapor can be adsorbed at low relative pressure. The relative pressure ($P/P_0$) at the point where the gradient of the adsorption-side isotherm curve is maximum is preferably 0.15 or more, and is preferably 0.3 or less.

(b) Adsorption amount ratio L on a side of low relative pressure

The adsorption amount ratio L on a side of low relative pressure obtained by the following formula (1) is also preferably 1.3 or more.

$$L=(A_{0.3}-A_{0.1})/(A_{0.9}-A_{0.3})\ ...(1)$$

(in the formula, $A_{0.1}$ represents an adsorption amount (mL(STP)·g$^{-1}$) at a relative pressure ($P/P_0$) of 0.1, $A_{0.3}$ represents an adsorption amount (mL(STP)·g$^{-1}$) at a relative pressure ($P/P_0$) of 0.3, and $A_{0.9}$ represents an adsorption amount (mL(STP)·g$^{-1}$) at a relative pressure ($P/P_0$) of 0.9, on the adsorption-side isotherm)

Thus, the adsorption amount on the side of low relative pressure can be made larger than that on the side of high relative pressure. The value of the above-described L is preferably 2.0 or more, more preferably 3.0 or more, and further preferably 5.0 or more. The upper limit of the value of L is not particularly limited, and the value of L may be, for example, 9.0 or less, or 8.0 or less.

(c) Adsorption amount at the relative pressure ($P/P_0$) of 0.3 on the adsorption-side isotherm

The adsorption amount at the relative pressure ($P/P_0$) of 0.3 on the adsorption-side isotherm is preferably 200 mL(STP)·g$^{-1}$ or more, more preferably 300 mL(STP)·g$^{-1}$ or more, and further preferably 350 mL(STP)·g$^{-1}$ or more. The upper limit thereof is not particularly limited, and the adsorption amount may be, for example, 800 mL(STP)·g$^{-1}$ or less, or 700 mL(STP)·g$^{-1}$ or less.

(d) Amount of change in the water vapor adsorption amount in the section where the adsorption-side isotherm rises with an inclination of 1000 mL(STP)·g$^{-1}$/relative pressure ($P/P_0$) or more

The amount of change in the water vapor adsorption amount in the section where the adsorption-side isotherm rises with the inclination of 1000 mL(STP)·g$^{-1}$/relative pressure ($P/P_0$) or more is a value of the difference between the maximum value and the minimum value of the adsorption amount, in the section where the inclination of the adsorption-side isotherm satisfies the condition of being 1000 mL(STP)·g$^{-1}$/relative pressure ($P/P_0$) or more. The amount of change is preferably 200 mL(STP)·g$^{-1}$ or more, more preferably 250 mL(STP)·g$^{-1}$ or more, and further preferably 350 mL(STP)·g$^{-1}$ or more. The upper limit thereof is not particularly limited, and the amount of change may be 600 mL(STP)·g$^{-1}$ or less or 500 mL(STP)·g$^{-1}$ or less.

(e) Relative pressure hysteresis ΔH

The relative pressure hysteresis ΔH obtained by the following formula (2) is preferably within ±0.03.

$$\Delta H = AH - DH \ \ldots(2)$$

(in the formula, AH represents an average value ((P1+P2)/2) of a minimum relative pressure $(P/P_0)$ P1 and a maximum relative pressure $(P/P_0)$ P2 in the section where the inclination is 1000 mL(STP)·$g^{-1}$/relative pressure $(P/P_0)$ or more on the adsorption-side isotherm, and DH represents an average value ((P3+P4)/2) of a minimum relative pressure $(P/P_0)$ P3 and a maximum relative pressure $(P/P_0)$ P4 in the section where the inclination is 1000 mL(STP)·$g^{-1}$/relative pressure $(P/P_0)$ or more on a desorption-side isotherm)

When the humidity hysteresis ΔH is in the above range, water that has been adsorbed can be efficiently desorbed, and the ratio of a desorption amount to an adsorption amount, as evaluated in Examples described below, can be increased, for example. ΔH is preferably within ±0.025, more preferably within ±0.02, and even more preferably within ±0.01. ΔH may be 0.002 or more or -0.002 or less.

(f) Adsorption amount hysteresis ΔA

The adsorption amount hysteresis ΔA obtained by formula (3) is preferably within ±30 mL(STP)·$g^{-1}$.

$$\Delta A = AA - DA \ \ldots(3)$$

(in the formula, AA represents an average value ((A1+A2)/2) of an adsorption amount A1 (mL(STP)·$g^{-1}$) at a minimum relative pressure $(P/P_0)$ and an adsorption amount A2 (mL(STP)·$g^{-1}$) at a maximum relative pressure $(P/P_0)$ in the section where the inclination is 1000 mL(STP)·$g^{-1}$/relative pressure $(P/P_0)$ or more on the adsorption-side isotherm, and DA represents an average value ((A3+A4)/2) of an adsorption amount A3 (mL(STP)·$g^{-1}$) at a minimum relative pressure $(P/P_0)$ and an adsorption amount A4 (mL(STP)·$g^{-1}$) at a maximum relative pressure $(P/P_0)$ in the section where the inclination is 1000 mL(STP)·$g^{-1}$/relative pressure $(P/P_0)$ or more on a desorption-side isotherm)

When the adsorption amount hysteresis ΔA is within the above range, same as described above for the humidity hysteresis ΔH, water that has been adsorbed can be efficiently desorbed, and the ratio of the desorption amount to the adsorption amount, as evaluated in Examples described below, can be increased, for example. ΔA is preferably within ±27 mL(STP)·$g^{-1}$, and more preferably within ±20 mL(STP)·$g^{-1}$. ΔA may be 3 mL(STP)·$g^{-1}$ or more, 5 mL(STP)·$g^{-1}$ or more, or -3 mL(STP)·$g^{-1}$ or less.

As for the MOF of the present invention, as long as the inclination of the tangent line drawn at the point where the gradient of the curve is maximum is 7000 mL(STP)·$g^{-1}$/relative pressure $(P/P_0)$ or more, and the water vapor adsorption amount at a relative pressure $(P/P_0)$ of 0.96 is 440 mL(STP)·$g^{-1}$ or more on the adsorption-side isotherm of water vapor measured at 25°C, a metal and an organic ligand constituting the MOF are not particularly limited, and examples thereof are as follows.

A metal ion constituting the MOF is preferably an ion of at least one metal selected from elements in groups 2 to 14 in the third to sixth periods in the periodic table. In the present specification, the term "metal" is used to include metalloids such as Si and Ge. The metal ion constituting the MOF is preferably an ion of at least one metal selected from the group consisting of Al, Ga, In, Ti, Zr, Hf, V, Cr, Mn, Fe, Co, Ni, and Cu, and is more preferably an ion of at least one metal selected from the group consisting of Al, Ti, and Cu.

An organic ligand constituting the MOF preferably includes at least one selected from the group consisting of carboxylates (carboxylate) represented by $R(COO^-)_n$ (R represents an n-valent group, and n represents an integer of 2 or more) and an oxalate ion $(COO^-)_2$. R preferably represents an aliphatic chain hydrocarbon group, an aliphatic cyclic hydrocarbon group, an aliphatic heterocyclic hydrocarbon group (group in which one or more carbon atoms of an aliphatic cyclic hydrocarbon group are substituted with heteroatoms), an aromatic hydrocarbon group, or an aromatic heterocyclic hydrocarbon group (group in which one or more carbon atoms of an aromatic hydrocarbon group are substituted with heteroatoms). The aliphatic chain hydrocarbon group may be a linear chain structure or a branched chain structure, and may be a saturated hydrocarbon group or an unsaturated hydrocarbon group. A heteroatom in the aliphatic heterocyclic hydrocarbon group or the aromatic heterocyclic hydrocarbon group is preferably nitrogen.

n is 2 or more, preferably 4 or less, more preferably 2 or more and 3 or less, and most preferably 2.

The above-described aliphatic chain hydrocarbon group, aliphatic cyclic hydrocarbon group, aliphatic heterocyclic hydrocarbon group, aromatic hydrocarbon group, and aromatic heterocyclic hydrocarbon group further include one or more functional groups X selected from the group consisting of a carboxylic acid anhydride group, -OH, $-OR^1$, $-NH_2$, $-NHR^1$, $-N(R^1)_2$, -CN, a halogeno group, -C(=S)SH, -C(=O)SH and a tautomer thereof, and $-SO_3H$. Each $R^1$ represents an

alkyl group having 1 or 2 carbon atoms. As the above functional group X, -OH or $-NH_2$ is particularly preferable.

Examples of the aromatic heterocyclic hydrocarbon group include pyrazole, imidazole, thiazole, oxazole, pyridine, pyrimidine, pyridazine, pyrazine, and triazine, and pyrazole is particularly preferable.

R preferably represents at least one of an unsaturated linear hydrocarbon group, an aromatic hydrocarbon group, and a hydrocarbon group containing an aromatic ring that includes a nitrogen atom, which may have the above-described functional group X.

The number of carbon atoms of R is preferably 1 or more, and more preferably 2 or more, and is preferably 18 or less, more preferably 12 or less, and further preferably 10 or less.

In particular, the organic ligand constituting the MOF preferably includes at least one selected from the group consisting of carboxylates represented by $R(COO^-)_2$ and an oxalate ion $(COO^-)_2$, and, specifically, examples thereof include a ligand in which two protons are removed from two carboxyl groups (-COOH) of the following dicarboxylic acid. Examples of the dicarboxylic acid include oxalic acid, succinic acid, fumaric acid, tartaric acid, 1,4-butanedicarboxylic acid, 1,4-butenedicarboxylic acid, 4-oxopyran-2,6-dicarboxylic acid, 1,6-hexanedicarboxylic acid, decanedicarboxylic acid, 1,8- heptadecanedicarboxylic acid, 1,9-heptadecanedicarboxylic acid, heptadecanedicarboxylic acid, acetylenedicarboxylic acid, 1,2-benzene dicarboxylic acid (phthalic acid), 1,3-benzene dicarboxylic acid (isophthalic), 2,3-pyridine dicarboxylic acid, 1,3-butadiene-1,4-dicarboxylic acid, 1,4-benzene dicarboxylic acid (terephthalic acid), 2-aminoterephthalic acid, 2,5-dihydroxyterephthalic acid, imidazole-2,4-dicarboxylic acid, 3,5-pyrazole dicarboxylic acid, 2-methylquinoline-3,4-dicarboxylic acid, quinoline-2,4-dicarboxylic acid, quinoxaline-2,3-dicarboxylic acid, 6-chloroquinoxaline-2,3-dicarboxylic acid, 4,4'-diaminodiphenylmethane-3,3'-dicarboxylic acid, quinoline-3,4-dicarboxylic acid, 7-chloro-4-hydroxyquinoline-2,8-dicarboxylic acid, diimide dicarboxylic acid, pyridine-2,6-dicarboxylic acid, 2-methylimidazole-4,5-dicarboxylic acid, thiophene-3,4-dicarboxylic acid, 2-isopropylimidazole-4,5-dicarboxylic acid, tetrahydropyran-4,4-dicarboxylic acid, perylene-3,9-dicarboxylic acid, perylene dicarboxylic acid, Pluriol E 200-dicarboxylic acid, 3,6-dioxaoctane dicarboxylic acid, 3,5-cyclohexadiene-1,2-dicarboxylic acid, octane dicarboxylic acid, pentane-3,3-carboxylic acid, 4,4'-dianiino-1,1'-biphenyl-3,3'-dicarboxylic acid, 4,4'-diaminobiphenyl-3,3'-dicarboxylic acid, benzidine-3,3'-dicarboxylic acid, 1,4-bis(phenylamino)benzene-2,5-dicarboxylic acid, 1,1'-binaphthyl dicarboxylic acid, 7-chloro-8-methylquinoline-2,3-dicarboxylic acid, 1-anilino anthraquinone-2,4'-dicarboxylic acid, polytetrahydrofuran 250-dicarboxylic acid, 1,4-bis(carboxymethyl) piperazine-2,3-dicarboxylic acid, 7-chloroquinotine-3,8-dicarboxylic acid, 1-(4-carboxy)phenyl-3-(4-chloro) phenylpyrazoline-4,5-dicarboxylic acid, 1,4,5,6,7,7-hexachloro-5-norbornene-2,3-dicarboxylic acid, phenylindan dicarboxylic acid, 1,3-dibenzyl-2-oxoimidazolidine-4,5-dicarboxylic acid, 1,4-cyclohexanedicarboxylic acid, naphthalene-1,8-dicarboxylic acid, 2-benzoylbenzene-1,3-dicarboxylic acid, 1,3-dibenzyl-2-oxoimidazolidine-4,5-cis-dicarboxylic acid, 2,2'-biquinoline-4,4'-dicarboxylic acid, pyridine-3,4-dicarboxylic acid, 3,6,9-trioxaundecanedicarboxylic acid, hydroxybenzophenone dicarboxylic acid, Pluriol E 300-dicarboxylic acid, Pluriol E 400-dicarboxylic acid, Pluriol E 600-dicarboxylic acid, pyrazole-3,4-dicarboxylic acid, 2,3-pyrazine dicarboxylic acid, 5,6-dimethyl-2,3-pyrazine dicarboxylic acid, bis(4-aminophenyl)ether diimide-dicarboxylic acid, 4,4'-diaminodiphenylmethane diimide-dicarboxylic acid, bis(4-aminophenyl)sulfone diimide-dicarboxylic acid, 1,4-naphthalene dicarboxylic acid, 2,6-naphthalene dicarboxylic acid, 1,3-adamantane dicarboxylic acid, 1,8-naphthalene dicarboxylic acid, 2,3-naphthalene dicarboxylic acid, 8-methoxy-2,3-naphthalene dicarboxylic acid, 8-nitro-2,3-naphthalene dicarboxylic acid, 8-sulfo-2,3-naphthalene dicarboxylic acid, anthracene-2,3-dicarboxylic acid, 2',3'-diphenyl-p-terphenyl-4,4''-dicarboxylic acid, (diphenyl ether)-4,4'-dicarboxylic acid, imidazole-4,5-dicarboxylic acid, 4(1H)-oxothiochromene-2,8-dicarboxylic acid, 5-tert-butyl-1,3-benzene dicarboxylic acid, 7,8-quinoline dicarboxylic acid, 4,5-imidazole dicarboxylic acid, 4-cyclohexene-1,2-dicarboxylic acid, hexatriacontane dicarboxylic acid, tetradecanedicarboxylic acid, 1,7-heptanedicarboxylic acid, 5-hydroxy-1,3-benzene dicarboxylic acid, 2,5-dihydroxy-1,4-benzene dicarboxylic acid, pyrazine-2,3-dicarboxylic acid, furan-2,5-dicarboxylic acid, 1-nonene-6,9-dicarboxylic acid, eicosene dicarboxylic acid, 4,4'-dihydroxy-diphenylmethane-3,3'-dicarboxylic acid, 1-amino-4-methyl-9,10-dioxo-9,10-dihydroanthracene-2,3-dicarboxylic acid, 2,5-pyridine dicarboxylic acid, cyclohexene-2,3-dicarboxylic acid, 2,9-dichlorofluorubine-4,11-dicarboxylic acid, 7-chloro-3-methylquinoline-6,8-dicarboxylic acid, 2,4-dichlorobenzophenone-2',5'-dicarboxylic acid, 1,3-benzene dicarboxylic acid, 2,6-pyridine dicarboxylic acid, 1-methylpyrrole-3,4-dicarboxylic acid, 1-benzyl-1H-pyrrole-3,4-dicarboxylic acid, anthraquinone-1,5-dicarboxylic acid, 3,5-pyrazole dicarboxylic acid, 2-nitrobenzene-1,4-dicarboxylic acid, heptane-1,7-dicarboxylic acid, cyclobutane-1,1-dicarboxylic acid, 1,14-tetradecanedicarboxylic acid, 5,6-dehydronorbornane-2,3-dicarboxylic acid, 5-ethyl-2,3-pyridine dicarboxylic acid, and camphordicarboxylic acid. The dicarboxylic acid is preferably at least one selected from the group consisting of fumaric acid, isophthalic acid, 2-aminoterephthalic acid, 2,5-dihydroxyterephthalic acid, and 3,5-pyrazole dicarboxylic acid.

As the organic ligand constituting the MOF of the present invention, a different type of an organic ligand other than carboxylates represented by $R(COO^-)_n$ and an oxalate ion $(COO^-)_2$ may be further included, and examples of the organic ligand include at least one selected from the group consisting of urea, pyrazine, oxazole, isoxazole, thiazole, imidazole, pyrazole, 1,2,3-thiadiazole, pyridazine, pyrimidine, purine, and pteridine.

In addition, the MOF of the present invention may include an inorganic ligand, and examples of the inorganic ligand include $OH^-$ (hydroxide), $O^{2-}$ (oxo), and $OH_2$ (aqua).

The molar ratio of the metal ion to the organic ligand (total amount thereof, in a case of a plurality of kinds of organic ligands) (metal ion/organic ligand) is preferably 0.05 or more, more preferably 0.1 or more, further preferably 0.2 or more, and even more preferably 0.5 or more, and the molar ratio is preferably 10 or less, more preferably 8 or less, and further preferably 5 or less.

In particular, the molar ratio of the metal ion to the total of the carboxylates represented by $R(COO^-)_n$ and an oxalate ion $(COO^-)_2$, among organic ligands, (metal ion/organic ligands) is preferably 0.2 or more, more preferably 0.5 or more, and further preferably 1 or more, and the molar ratio is preferably 15 or less, more preferably 10 or less, and further preferably 8 or less.

The BET specific surface area of the MOF is preferably 495 $cm^2/g$ or more, more preferably 600 $cm^2/g$ or more, and further preferably 700 $cm^2/g$ or more. The upper limit of the BET specific surface area is not particularly limited, and the BET specific surface area is, for example, 2000 $cm^2/g$ or less.

Next, a method for producing the MOF of the present invention will be described. The MOF of the present invention can be obtained by reacting, in a solvent, a metal compound containing the metal ion constituting the MOF and one or more kinds of organic compounds for the organic ligands constituting the MOF. In addition, an inorganic compound for the inorganic ligands is also preferably mixed with the solvent together with the metal compound and the organic compounds, as necessary.

The metal compound that contains the metal ion constituting the MOF is preferably metal sulfate, metal acetate, metal chloride, or metal alkoxide.

The organic compound for the organic ligands constituting the MOF is preferably one or more selected from the group consisting of polyvalent carboxylic acids represented by $R(COOH)_n$ and oxalic acid. R and n are equivalent to R and n described above for the carboxylates represented by $R(COO^-)_n$, and all the above descriptions about R and n, also including preferable aspects thereof, can be referred to.

The organic compound preferably includes dicarboxylic acid. For specific examples of the dicarboxylic acid, the dicarboxylic acids described for the carboxylates represented by $R(COO^-)_n$ and preferable ranges thereof can be referred to.

As the organic compound for the organic ligands, in addition to one or more selected from the group consisting of the polyvalent carboxylic acids represented by $R(COOH)_n$ and oxalic acid, at least one selected from the group consisting of urea, pyrazine, oxazole, isoxazole, thiazole, imidazole, pyrazole, 1,2,3-thiadiazole, pyridazine, pyrimidine, purine, and pteridine is preferably used. In addition, as the above-described inorganic compound for the inorganic ligands, alkali metal hydroxide is preferably used. Furthermore, in some cases, an inorganic ligand such as $OH^-$, $O^{2-}$, or $OH_2$ derived from the solvent for dissolving the metal compound and the organic compound, water in the air, and the like is included in the MOF.

The solvent is not particularly limited as long as the solvent is capable of dissolving the metal compound, the organic compound for the organic ligands, and the inorganic compound for the inorganic ligands to be used as necessary, and an appropriate solvent can be selected from water and an organic solvent. The solvent is preferably water, an alcohol-based solvent such as methanol, or an amide-based solvent such as dimethylformamide.

The ratio of the metal compound to the solvent (metal compound/solvent) is preferably 0.005 mol/L or more, more preferably 0.01 mol/L or more, and further preferably 0.05 mol/L or more, and the ratio is preferably 5 mol/L or less, more preferably 3 mol/L or less, and further preferably 2 mol/L or less.

The ratio of the organic compound for the organic ligands to the solvent (organic compound/solvent) is preferably 0.001 mol/L or more, more preferably 0.1 mol/L or more, and further preferably 0.2 mol/L or more, and the ratio is preferably 3 mol/L or less, more preferably 2 mol/L or less, and further preferably 1 mol/L or less. The ratio of (the total amount of) the polyvalent carboxylic acids, represented by $R(COOH)_n$ and oxalic acid, as a preferable aspect of the organic compound for the organic ligand, to the solvent is preferably 0.001 mol/L or more, more preferably 0.1 mol/L or more, and further preferably 0.2 mol/L or more, and the ratio is preferably 2 mol/L or less, more preferably 1 mol/L or less, and further preferably 0.5 mol/L or less.

The ratio of the inorganic compound for the inorganic ligands to the solvent (inorganic compound/solvent) is preferably 0.01 mol/L or more, and more preferably 0.05 mol/L or more, and the ratio is preferably 3 mol/L or less, more preferably 2 mol/L or less, and further preferably 1 mol/L or less.

The molar ratio between the metal compound and the organic compound may be adjusted such that the ratio of the molar quantity of metal atoms in the metal compound to the molar quantity of the organic compound is equal to the above-described molar ratio of the metal ion to the organic ligand.

In order to obtain the MOF of the present invention, it is important to appropriately adjust, particularly, at least any of: (i) the order of mixing of the metal compound and the organic compound for the organic ligand; (ii) the temperature of stirring or being left as they are, after the metal compound and the organic compound for the organic ligand are mixed; (iii) a post-treatment condition (washing condition) for the reaction product of the metal compound and the organic compound for the organic ligand; and (iv) a drying condition after the washing.

Regarding (i), in mixing of the metal compound and the organic compound for the organic ligand, it is important that the metal compound and the organic compound are completely dissolved. Specifically, it is preferable that one of the metal compound and the organic compound is completely dissolved in the solvent and then the other is added (at this time, it is preferable that the other is also completely dissolved in the solvent), and it is preferable that the metal compound is added and also completely dissolved in a liquid of the organic compound which has been completely dissolved in the solvent, or that a liquid of the metal compound which has been completely dissolved in the solvent is added to a liquid of the organic compound which has been completely dissolved in the solvent.

In the case where the MOF of the present invention further includes, in addition to the metal ion and at least one kind of the organic ligand selected from the group consisting of $R(COO^-)_n$ and an oxalate ion $(COO^-)_2$, a different type of an inorganic ligand and/or an organic ligand other than $R(COO^-)_n$ and an oxalate ion $(COO^-)_2$, it is preferable that the metal compound is added to a liquid including such ligands or that such ligands are added at the same time when the metal compound is added.

Regarding (ii), after the metal compound and the organic compound for the organic ligand are mixed, specifically, the obtained mixture may be stirred and/or left as they are at a temperature from room temperature (for example, 25°C) to 170°C for 5 minutes to 40 hours (preferably, 10 to 40 hours).

Regarding (iii), in the post-treatment condition for the reaction product of the metal compound and the organic compound for the organic ligand, specifically, it is preferable that the reaction product is washed (for example, three times or more) with a solvent same as the solvent used during the reaction of the metal compound and the organic compound, and it is also preferable that the washed reaction product is, thereafter, further washed with an alcohol-based solvent.

Regarding (iv), the drying may be performed under reduced pressure at a temperature of 80 to 170°C for 10 to 100 hours, or in an air atmosphere at a temperature of 25 to 150°C for 20 to 100 hours. The temperature for drying under reduced pressure is preferably 100 to 170°C, and the time for drying under reduced pressure is preferably 10 to 50 hours, and more preferably 24 to 48 hours. The temperature and the time may be optionally combined within the above ranges.

The MOF of the present invention can be suitably used to adsorb and remove gas or organic molecules, for example. Examples of the gas include carbon dioxide, hydrogen, carbon monoxide, oxygen, nitrogen, hydrocarbon having 1 to 4 carbon atoms, noble gas, hydrogen sulfide, ammonia, sulfur oxide, nitrogen oxide, and siloxane. Examples of the organic molecule include hydrocarbon having 5 to 8 carbon atoms, alcohol having 1 to 8 carbon atoms, aldehyde having 1 to 8 carbon atoms, carboxylic acid having 1 to 8 carbon atoms, ketone having 1 to 8 carbon atoms, amine having 1 to 8 carbon atoms, ester having 1 to 8 carbon atoms, and amide having 1 to 8 carbon atoms. The organic molecule may contain an aromatic ring.

This application claims the benefit of priority based on the Japanese Patent Application No. 2022-042376 filed on March 17, 2022. The entire contents of the Japanese Patent Application No. 2022-042376 filed on March 17, 2022, are incorporated herein by reference.

EXAMPLE

The present invention will be described in more detail below by means of examples. The present invention. The present invention is not limited by the following examples, and can also be carried out with appropriate modifications being made within the scope of the gist described above and below, and any of these modifications are included in the technical scope of the present invention.

Example 1

In a 50 mL pressure-resistant container (including a Teflon (registered trademark) inner cylinder) made of SUS-304, 3.000 mmol of 2-aminoterephthalic acid was dissolved in 25 mL of a mixture obtained by mixing DMF and MeOH at a ratio of 1/1 (volume ratio), and 1.518 mmol of $Ti[OCH(CH_3)_2]_4$ was mixed with the resulting product at 25°C, and the obtained mixture was stirred for 5 minutes by putting in a stirrer chip to completely dissolve. Then, the resulting product was left as it was at 150°C for 16 hours. The obtained precipitated solid product was subjected to three times of washing with 10 mL of DMF and filtering, and the obtained filter cake was dried in a vacuum drying oven at 120°C for 24 hours and at 150°C for 24 hours, to obtain 0.62 g of a product (yield: 89.4%).

Example 2

In a 20 mL eggplant flask, 3.233 mmol of isophthalic acid was mixed with 2.5 mL of DMF at 25°C and completely dissolved, to obtain a solution A. Separately, 3.693 mmol of $Al_2(SO_4)_3 \cdot nH_2O$ (n=14 to 18) and 10 mL of ion-exchanged water were mixed and $Al_2(SO_4)_3 \cdot nH_2O$ was completely dissolved to prepare a solution B, and the solution B was added dropwise to the solution A at 25°C over 5 minutes. Then, the resulting solution was refluxed at 125°C for 24 hours, to obtain a suspension. Decantation was performed on the suspension. The precipitated solid product was separated by centrifugation and washed three times with 10 ml of water. The obtained filter cake was dried in a vacuum drying oven at 80°C for 24 hours, at 100°C for 24 hours, and at 120°C for 48 hours, to obtain 0.66 g of a product (yield: 99%).

Example 3

In a 100 mL pressure-resistant container (including a Teflon (registered trademark) inner cylinder) made of SUS-304, 6.226 mmol of fumaric acid, 6.402 mmol of urea, and 30.549 mmol of $Al_2(SO_4)_3 \cdot nH_2O$ (n=14 to 18) were added to 20 ml of ion-exchanged water in this order, and were mixed at 25°C for 10 minutes, to be completely dissolved. Then, the resulting product was left as it was at 110°C for 32 hours. The obtained precipitated solid product was subjected to three times of washing with 30 ml of ethanol and filtering, and the obtained filter cake was dried at 25°C for 72 hours, to obtain 0.91 g of a product (yield: 23%).

Example 4

In a 100 mL pressure-resistant container (including a Teflon (registered trademark) inner cylinder) made of SUS-304, 4.43 mmol of 3,5-pyrazole dicarboxylic acid monohydrate was mixed with 72 ml of ion-exchanged water and completely dissolved, to obtain a solution A. Separately, 6.580 mmol of $LiOH \cdot H_2O$ was mixed with 2.4 ml of ion-exchanged water, and was completely dissolved by applying ultrasonic waves at 50°C, to obtain a solution B. The solution B was added to the solution A, and 4.333 mmol of $AlCl_3 \cdot 6H_2O$ and 53.017 mmol of pyrazine were further added in this order, and were mixed at 25°C for 5 minutes. Then, the resulting product was left as it was at 100°C for 15 hours. The obtained precipitated solid product was subjected to three times of washing with ion-exchanged water and filtering (60 ml of ion-exchanged water was used for washing in total), and was further subjected to three times of washing with methanol and filtering (60 ml of methanol was used for washing in total). The obtained filter cake was dried in a vacuum drying oven at 100°C for 24 hours, to obtain 0.52 g of a product (yield: 41.8%).

Example 5

In a 20 mL eggplant flask, 2.123 mmol of fumaric acid and 6.39 mmol of NaOH were mixed with 3 ml of ion-exchanged water, to obtain a solution A in which the fumaric acid and NaOH were completely dissolved. Separately, 1.064 mmol of $Al_2(SO_4)_3 \cdot 18H_2O$ was mixed with 3.61 ml of ion-exchanged water and completely dissolved, to obtain a solution B. The solution B was added to the solution A at 60°C over 25 minutes, and the resulting solution was stirred at 60°C for 5 minutes. The obtained precipitated solid product was subjected to three times of washing with ion-exchanged water and filtering (50 ml of ion-exchanged water was used for washing in total), and the obtained filter cake was dried in an oven in air at 100°C for 24 hours and at 130°C for 24 hours, to obtain 0.14 g of a product (yield: 99%).

Example 6

In a 200 mL eggplant flask, 2.071 mmol of $Cu(OAc)_2 \cdot H_2O$ was mixed with 168 ml of methanol and completely dissolved, to obtain a solution A. Separately, 1.014 mmol of 2,5-dihydroxyterephthalic acid was mixed with 16.8 ml of methanol and completely dissolved, to obtain a solution B. The solution B was added to the solution A at 25°C over 60 minutes, and the resulting solution was stirred at 25°C to 30°C for 12 hours. The obtained precipitated solid product was separated by centrifugation, and then was washed five times with 15 ml of methanol, and the obtained cake was dried in a vacuum drying oven at 120°C for 24 hours, to obtain 0.30 g of a product (yield: 92.1%).

Comparative Example 1

In a 50 mL pressure-resistant container (including a Teflon (registered trademark) inner cylinder) made of SUS-304, 1.058 mmol of InCh and 8 ml of DMF were mixed, 2.081 mmol of terephthalic acid was added thereto, and the resulting mixture was stirred at 25°C for 8 hours to form a gel. Then, the resulting mixture was left as it was at 160°C for 3 days. The obtained solid product was separated by centrifugation, and then was washed five times with 15 ml of methanol, and the obtained cake was dried in a vacuum drying oven at 120°C for 24 hours, to obtain 0.46 g of a product (yield: 99%).

Comparative Example 2

In a 100 mL pressure-resistant container (including a Teflon (registered trademark) inner cylinder) made of SUS-304, 0.5 mmol of terephthalic acid, 3.691 mmol of $Al(NO_3)_3 \cdot 9H_2O$, and 40.2 ml of methanol were mixed, 1.8 ml of a 2 M solution of NaOH in methanol was added thereinto at room temperature, and the resulting mixture was mixed at 25°C for 5 minutes. Then, the resulting mixture was left as it was at 125°C for 20 hours. The obtained precipitated solid product was subjected to three times of washing with 30 ml of methanol and filtering, and the obtained filter cake was dried at room temperature for 72 hours, to obtain 0.17 g of a product (yield: 99%).

The substances obtained in Examples and Comparative Examples were evaluated by the following methods.

(1) Ratio of desorption amount to adsorption amount

Device: TG-DTA manufactured by Rigaku Corporation

[Measurement of desorption amount]

Pre-treatment condition: each substance was held at 25°C for 12 hours under an air atmosphere in which the humidity was adjusted so that the relative pressure was 0.5.

Desorbed amount: after the above pre-treatment, the temperature was increased at a heating rate of 5°C/min. while nitrogen was caused to flow, and the substance was held at 50°C for 30 minutes. A weight reduction amount $W_{25-50}$ in this range (25 to 50°C) was measured and $W_{25-50}$ was defined as a desorption amount.

[Measurement of adsorption amount]

After the desorption amount was measured, the temperature was further increased at a heating rate of 5°C/min. while nitrogen was caused to flow at 25°C at a relative pressure of 0.5, and the substance was held at 200°C for 1 hour. A weight reduction amount $W_{50-200}$ in this range (50 to 200°C) was measured, and the total of the amount $W_{25-50}$ and the amount $W_{50-200}$ was defined as an adsorption amount.

The ratio (%) of the desorption amount to the adsorption amount as measured according to the above procedure was obtained.

(2) Adsorption and desorption isotherms of water vapor

Pre-treatment condition:

(i) Each sample was heated from 100°C to a temperature that was 50°C lower than a decomposition start temperature of the MOF, and was dried under reduced pressure for 12 hours;
(ii) The sample tube was connected to a pre-treatment device (BELPREP VACII), the air inside the sample tube was discharged, and then, $N_2$ gas (having a purity of 99.999% or more) was introduced therein until the pressure reached atmospheric pressure;
(iii) Thereafter, the sample tube was removed from the pre-treatment device, and the weight was measured by using a precision balance (that displays four or more decimal places) three times to obtain an average value (W1). When the precision balance was used, an ionizer was used to eliminate the effect of static electricity;
(iv) About 50 mg of the sample to be measured was weighed, and the sample was directly placed into a spherical part in the lower part of a standard sample tube by using a long stem funnel;
(v) A glass bar was returned into the sample tube, the sample tube was sealed with a quick seal, and then the total weight was measured once and the amount of the added sample was tentatively checked. The sample tube containing the sample was connected to the pre-treatment device to exhaust gas from the sample tube; and
(vi) After the pressure in the sample tube became sufficiently low, heating was started (while vacuuming was continued).

Measurement device: BELSORP-max manufactured by MicrotracBEL
Measurement condition:

(i) A water adsorption amount as a weight percent (wt%) was calculated as (water adsorption amount/(amount of measurement sample)×100), by inputting the weight of the sample and information on water at a measurement temperature (saturation vapor pressure, and the like) to the measurement software, setting the measurement relative pressure, at which measurement was desired to be performed, and the like, and starting the measurement;
(ii) Ultrapure water which was an adsorbate was placed into a solvent reservoir, and the solvent reservoir was connected to the device. Then, defoaming treatment was performed thereon, and the solvent reservoir was immersed in liquid nitrogen filled in a Dewar vessel to freeze the ultrapure water;
(iii) After it was confirmed that the ultrapure water was sufficiently frozen, a valve in the upper part of the solvent reservoir was opened to evacuate the solvent reservoir. The valve in the upper part of the solvent reservoir was closed. The frozen water was melted, and the air contained in the ultrapure water was taken out as gas at least three times, to complete preparation of the adsorbate;

(iv) The weight of the sample, and information on water at a measurement temperature (saturation vapor pressure, and the like) were inputted to the measurement software, the measurement relative pressure, at which measurement was desired to be performed, and the like was set, and a measurement start button was pressed; and

(v) Thereafter, according to instructions of the software, the Dewar vessel filled with liquid nitrogen or the sample tube was set, and measurement was performed.

(3) Measurement of BET specific surface area

Since the adsorption occupation area of nitrogen molecules is known in advance, the amount of gas molecules adsorbed on only the surface of the sample was measured, and the surface area of the sample was measured according to a BET adsorption isotherm.

Pre-treatment condition: according to the measurement results from TG-DTA described above, the temperature at which water contained in the sample was able to be removed was checked, and heating under reduced pressure was performed overnight.

Device: BELSORP-mini manufactured by MicrotracBEL

Pre-treatment condition: (i) A glass bar (for a standard sample tube) for reducing the volume was set in a standard sample tube, and the standard sample tube was sealed with a quick seal. The above sample-tube sets, the number of which corresponded to the number of samples to be measured (a maximum of 3 samples per measurement), were prepared and connected to a pre-treatment device (BELPREP VACII). The air inside the sample tube was discharged, and then Nz gas (having a purity of 99.999% or more) was introduced therein until the pressure reached atmospheric pressure;

(ii) Thereafter, the sample tube was removed from the pre-treatment device, and the weight was measured by using a precision balance (that displays four or more decimal places) three times to obtain the average value (W1). When the precision balance was used, an ionizer was used to eliminate the effect of static electricity;

(iii) About 50 mg of the sample to be measured was weighed on drug packaging paper, and the sample was directly placed into a spherical part in the lower part of the standard sample tube by using a long stem funnel;

(iv) The glass bar was returned into the sample tube, the sample tube was sealed with a quick seal, and then the total weight was measured once and the amount of the added sample was tentatively checked;

(v) The sample tube containing the sample was connected to the pre-treatment device to exhaust gas from the sample tube; and

(vi) After the pressure in the sample tube became sufficiently low, heating was started (while vacuuming was continued).

Measurement condition:

(i) After the pre-treatment (vacuum heating) was ended, heat was dissipated from the sample tube while the sample tube was held under reduced pressure. After the temperature reached room temperature, $N_2$ gas was introduced therein until the pressure reached atmospheric pressure. The sample tube was removed from the device;

(ii) After the pre-treatment, the weight of the sample tube containing the sample was measured three times by using a precision balance to obtain an average value (W2). The weight of the sample was obtained by performing calculation of W2-W1; and

(iii) The weight of the sample, and information on $N_2$ gas at a liquid nitrogen temperature (second virial coefficient, and the like) were inputted to the measurement software, the measurement relative pressure at which measurement was desired to be performed was inputted, and a measurement start button was pressed. Then, according to instructions of the software, a Dewar vessel filled with liquid nitrogen or a sample tube was set, and measurement was performed.

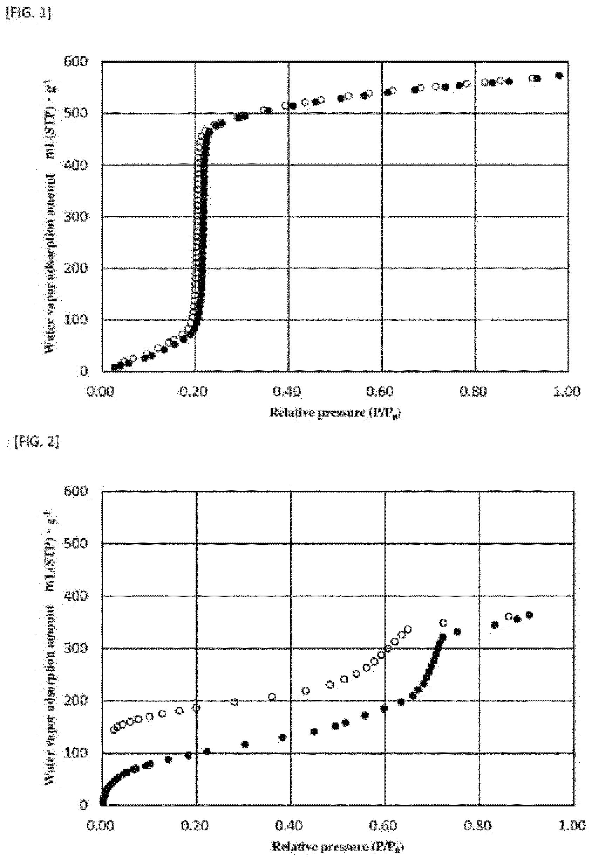

With respect to Examples and Comparative Examples, the inclination of a tangent line drawn at a point where the gradient of a curve is maximum, a relative pressure at the point where the gradient is maximum, water vapor adsorption amounts at relative pressures of 0.3 and 0.96, an adsorption amount ratio L on a side of low relative pressure, and the amount of change in the water vapor adsorption amount in the section where an adsorption-side isotherm rises with an inclination of 1000 mL(STP)·$g^{-1}$/relative pressure ($P/P_0$) or more, each of which is determined by adsorption and desorption isotherms of water vapor, as well as the results of the above-described (1) and (3), are shown in Table 1. Each organic ligand in Table 1 is indicated by the name of carboxylic acid in a state before protons are removed from carboxyl groups. In addition, the adsorption-side isotherms of Example 1 and Comparative Example 2 are respectively shown in FIG. 1 and FIG. 2. In FIGS. 1 and 2, • represents an adsorption-side isotherm, and o represents a desorption-side isotherm.

[Table 1]

| | Metal ion | Organic ligand | Other compound | Metal ion/Oranic ligand Molar ratio | Maximum inclination (mL·g⁻¹/relative pressure) | Relative pressure at the point of maximum inclination | Water vapor adsorption amount at a relative pressure of 0.3 (mL·g⁻¹) | Water vapor adsorption amount at a relative pressure of 0.96 (mL·g⁻¹) | Adsorption amount ratio L on a side of low relative pressure | Change in water vapor adsorption amount at the point of 1000 mL g⁻¹/relative pressure changes (mL·g⁻¹) | ΔH | ΔA (mL·g⁻¹) | BET specific surface area (cm²/g) | Ratio of desorption amount to adsorption amount (%) |
|---|---|---|---|---|---|---|---|---|---|---|---|---|---|---|
| Exapmle 1 | Ti | 2-aminoterephthalic acid | - | 0.5 | 33000 | 0.21 | 495 | 570 | 6.6 | 393 | 0.008 | 6 | 1388 | 49 |
| Exapmle 2 | Al | Isophthalic acid | - | 2.3 | 32500 | 0.17 | 371 | 445 | 5.1 | 333 | 0.013 | 8 | 664 | 90 |
| Exapmle 3 | Al | Fumaric acid | Urea | 4.8 | 9500 | 0.24 | 478 | 590 | 5.1 | 411 | 0.006 | 16 | 1153 | 93 |
| Exapmle 4 | Al | 3,5-pyrazole dicarboxylic acid monohydrate | Pyrazine LiOH·H₂O | 0.1 | 14800 | 0.11 | 489 | 580 or more | 3.8 | 309 | 0.006 | 17 | 749 | 82 |
| Exapmle 5 | Al | Fumaricacid | NaOH | 1.0 | 26300 | 0.25 | 366 | 500 | 2.9 | 272 | 0.020 | 27 | 959 | 50 |
| Exapmle 6 | Cu | 2,5-dihydroxyterephthalic acid | - | 2.0 | 8400 | 0.10 | 294 | 470 or more | 13 | 99 | 0.014 | 51 | 496 | 77 |
| Comparative example 1 | In | Terephthalic acid | - | 0.5 | 5200 | 0.96 | 59 | 330 | 0.2 | 216 | 0.041 | 4 | 292 | 22 |
| Comparative example 2 | Al | Terephthalic acid | NaOH | 7.4 | 2300 | 0.70 | 116 | 360 or more (less than 440) | 0.1 | 111 | 0.049 | 65 | 491 | 30 |

**Claims**

1. A metal organic framework, wherein

   on an adsorption-side isotherm of water vapor measured at 25°C, an inclination of a tangent line drawn at a point where a gradient of a curve is maximum is 7000 mL(STP)·g$^{-1}$/relative pressure (P/P$_0$) or more,
   a water vapor adsorption amount at a relative pressure (P/P$_0$) of 0.96 is 440 mL(STP)·g$^{-1}$ or more on the adsorption-side isotherm, and
   a vertical axis of the adsorption-side isotherm represents a water vapor adsorption amount and a horizontal axis of the adsorption-side isotherm represents a relative pressure (P/P$_0$) (P represents water vapor pressure and P$_0$ represents saturation water vapor pressure).

2. The metal organic framework according to claim 1, wherein the point where the gradient is maximum is in a range of relative pressures (P/P$_0$) of 0.1 to 0.4.

3. The metal organic framework according to claim 1 or 2, wherein
   an adsorption amount ratio L on a side of low relative pressure obtained by formula (1) is 1.3 or more.

$$L=(A_{0.3}-A_{0.1})/(A_{0.9}-A_{0.3}) \ldots (1)$$

   (in the formula, A$_{0.1}$ represents an adsorption amount (mL(STP)·g$^{-1}$) at a relative pressure (P/P$_0$) of 0.1, A$_{0.3}$ represents an adsorption amount (mL(STP)·g$^{-1}$) at a relative pressure (P/P$_0$) of 0.3, and A$_{0.9}$ represents an adsorption amount (mL(STP)·g$^{-1}$) at a relative pressure (P/P$_0$) of 0.9, on the adsorption-side isotherm)

4. The metal organic framework according to claim 1 or 2, wherein
   the adsorption amount at the relative pressure (P/P$_0$) of 0.3 on the adsorption-side isotherm is 200 mL(STP)·g$^{-1}$ or more.

5. The metal organic framework according to claim 1 or 2, wherein
   an amount of change in a water vapor adsorption amount in a section where the adsorption-side isotherm rises with an inclination of 1000 mL(STP)·g$^{-1}$/relative pressure (P/P$_0$) or more is 200 mL(STP)·g$^{-1}$ or more.

6. The metal organic framework according to claim 1 or 2, wherein
   a relative pressure hysteresis ΔH obtained by formula (2) is within ±0.03.

$$\Delta H = AH - DH \ldots (2)$$

   (in the formula, AH represents an average value ((PI+P2)/2) of a minimum relative pressure (P/P$_0$) P1 and a maximum relative pressure (P/P$_0$) P2 in a section where an inclination is 1000 mL(STP)·g$^{-1}$/relative pressure (P/P$_0$) or more on the adsorption-side isotherm, and DH represents an average value ((P3+P4)/2) of a minimum relative pressure (P/P$_0$) P3 and a maximum relative pressure (P/P$_0$) P4 in a section where an inclination is 1000 mL(STP)·g$^{-1}$/relative pressure (P/P$_0$) or more on a desorption-side isotherm)

7. The metal organic framework according to claim 1 or 2, wherein
   an adsorption amount hysteresis ΔA obtained by formula (3) is within ±30 mL(STP)·g$^{-1}$.

$$\Delta A = AA - DA \ldots (3)$$

   (in the formula, AA represents an average value ((A1+A2)/2) of an adsorption amount A1 (mL(STP)·g$^{-1}$) at a minimum relative pressure (P/P$_0$) and an adsorption amount A2 (mL(STP)·g$^{-1}$) at a maximum relative pressure (P/P$_0$) in a section where an inclination is 1000 mL(STP)·g$^{-1}$/relative pressure (P/P$_0$) or more on the adsorption-side isotherm, and DA represents an average value (A3+A4)/2) of an adsorption amount A3 (mL(STP)·g$^{-1}$) at a minimum relative pressure (P/P$_0$) and an adsorption amount A4 (mL(STP)·g$^{-1}$) at a maximum relative pressure (P/P$_0$) in a section where an inclination is 1000 mL(STP)·g$^{-1}$/relative pressure (P/P$_0$) or more on a desorption-side isotherm)

8. The metal organic framework according to claim 1 or 2, wherein

a metal ion constituting the metal organic framework is an ion of at least one metal selected from elements in groups 2 to 14 in the third to sixth periods in a periodic table.

9. The metal organic framework according to claim 1 or 2, wherein
an organic ligand constituting the metal organic framework includes at least one selected from the group consisting of carboxylates represented by $R(COO^-)_n$ (R represents an n-valent group, and n represents an integer of 2 or more) and an oxalate ion $(COO^-)_2$.

[FIG. 1]

[FIG. 2]

## INTERNATIONAL SEARCH REPORT

| International application No. |
|---|
| **PCT/JP2023/010263** |

**A. CLASSIFICATION OF SUBJECT MATTER**

***B01J 20/22*** (2006.01)i; ***B01D 53/28*** (2006.01)i; ***C07C 57/15*** (2006.01)i; ***C07C 63/24*** (2006.01)i; ***C07C 65/03*** (2006.01)i; ***C07C 229/76*** (2006.01)i; ***C07F 1/08*** (2006.01)i; ***C07F 5/06*** (2006.01)i; ***C07F 7/28*** (2006.01)i
FI: B01J20/22 A; B01D53/28; C07C57/15; C07C63/24; C07C65/03 D; C07C229/76; C07F1/08 B; C07F5/06 D; C07F7/28 F

According to International Patent Classification (IPC) or to both national classification and IPC

**B. FIELDS SEARCHED**

Minimum documentation searched (classification system followed by classification symbols)

B01J20/22; B01D53/28; C07C57/15; C07C63/24; C07C65/03; C07C229/76; C07F1/08; C07F5/06; C07F7/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

B01J20/00-20/34, B01D53/02-53/12, B01D53/26-53/28, C07C1/00-409/44, C07F1/00-19/00

**C. DOCUMENTS CONSIDERED TO BE RELEVANT**

| Category* | Citation of document, with indication, where appropriate, of the relevant passages | Relevant to claim No. |
|---|---|---|
| X | JP 2022-508231 A (THE REGENTS OF THE UNIVERSITY OF CALIFORNIA) 19 January 2022 (2022-01-19) claims, paragraphs [0007]-[0110], fig. 1-28 | 1-9 |
| X | JP 2018-172320 A (TOYOTA MOTOR CORP) 08 November 2018 (2018-11-08) claims, paragraphs [0007]-[0102], fig. 1-19 | 1-9 |
| X | US 2017/0341010 A1 (MASSACHUSETTS INSTITUTE OF TECHNOLOGY) 30 November 2017 (2017-11-30) paragraphs [0007]-[0154], fig. 1-12 | 1-9 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 May 2023** | **23 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

| INTERNATIONAL SEARCH REPORT Information on patent family members | | | International application No. PCT/JP2023/010263 |
|---|---|---|---|

| Patent document cited in search report | Publication date (day/month/year) | Patent family member(s) | Publication date (day/month/year) |
|---|---|---|---|
| JP 2022-508231 A | 19 January 2022 | US 2021/0283574 A1 claims, paragraphs [0007]-[0120], fig. 1-28<br>AU 2019386080 A1<br>BR 112021010139 A2<br>CA 3120865 A1<br>CL 2021001370 A1<br>CN 113272043 A<br>EP 3887022 A2<br>IL 283376 A<br>KR 10-2021-0105366 A<br>SG 11202105462P A<br>WO 2020/112899 A1<br>ZA 202103624 B | |
| JP 2018-172320 A | 08 November 2018 | US 2018/0280929 A1 claims, paragraphs [0007]-[0181], fig. 1-19<br>BR 102018006673 A2<br>CN 108690200 A<br>EP 3381924 A1<br>RU 2686901 C1 | |
| US 2017/0341010 A1 | 30 November 2017 | CN 109414688 A<br>EP 3463655 A1<br>WO 2017/205752 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020176101 A

- JP 2022042376 A